(19) Europäisches Patentamt
European Patent Office
Office européen des brevets

(11) **EP 1 240 009 B1**

(12) **EUROPEAN PATENT SPECIFICATION**

(45) Date of publication and mention
of the grant of the patent:
**18.11.2009 Bulletin 2009/47**

(51) Int Cl.:
**B32B 5/26** *(2006.01)*  **D04H 13/00** *(2006.01)*
**A61F 13/15** *(2006.01)*

(21) Application number: **00913512.0**

(22) Date of filing: **17.02.2000**

(86) International application number:
**PCT/US2000/004142**

(87) International publication number:
**WO 2001/030564 (03.05.2001 Gazette 2001/18)**

(54) **NONWOVEN COMPOSITE LAMINATE EMPLOYING NONWOVEN FORMED BY COMPONENT FIBERS OF ETHYLENE-PROPYLENE RANDOM COPOLYMER**

VERBUNDVLIESSTOFF DER EINEN VLIESSTOFF AUS FASERN AUS ETHYLEN-PROPYLEN-COPOLYMER MIT ZUFÄLLIGER VERTEILUNG BENUTZT

STRATIFIE COMPOSITE NON TISSE UTILISANT UN NON-TISSE FORME DE FIBRES DE COPOLYMERE ALEATOIRE D'ETHYLENE-PROPYLENE

(84) Designated Contracting States:
**AT BE CH CY DE DK ES FI FR GB GR IE IT LI LU MC NL PT SE**

(30) Priority: **22.10.1999 PCT/US99/24935**
**22.10.1999 PCT/US99/24936**

(43) Date of publication of application:
**18.09.2002 Bulletin 2002/38**

(73) Proprietor: **THE PROCTER & GAMBLE COMPANY**
**Cincinnati, Ohio 45202 (US)**

(72) Inventor: **OHNISHI, Kazuyuki**
**Takaishi-City,**
**Osaka 592-0003 (JP)**

(74) Representative: **Kremer, Véronique Marie Joséphine et al**
**Procter & Gamble Service GmbH**
**Sulzbacher Strasse 40-50**
**65824 Schwalbach am Taunus (DE)**

(56) References cited:
**EP-A- 0 683 260    WO-A-99/28544**
**WO-A-99/49120    WO-A-99/55942**
**US-A- 5 607 798    US-A- 5 652 051**

• **PATENT ABSTRACTS OF JAPAN vol. 1997, no. 03, 31 March 1997 (1997-03-31) & JP 08 302553 A (DAIWABO CO LTD), 19 November 1996 (1996-11-19)**

**Description**

FIELD

The present invention relates to nonwoven composite laminates. More specifically, the present invention relates to nonwoven composite laminates which employ a nonwoven layer formed by component fibers of an ethylene-propylene random copolymer. In combination with an insulation layer as defined in present claim 1. The present invention also relates to disposable articles using such a nonwoven composite laminate. Examples of such disposable articles include sweat bands, bandages, body wraps, disposable underwears, disposable garments including disposable diapers (adult and baby) including pull-on diapers and training pants, disposable panties for menstrual use, and disposable absorbent pads including sanitary napkins and incontinence devices.

BACKGROUND

Nonwoven composite laminates have previously been used in a variety of disposable products, including sweat bands, bandages, body wraps, disposable underwears, disposable garments including disposable diapers (adult and baby) including pull-on diapers and training pants, disposable panties for menstrual use, and disposable absorbent pads including sanitary napkins and incontinence devices.

For example, infants and other incontinent individuals wear disposable garments such as diapers to receive and contain urine and other body exudates. Disposable garments function to contain the discharged materials and to isolate these materials from the body of the wearer and from the wearer's garments and bed clothing. Disposable garments having many different basic designs are known to the art. Examples of such disposable garments include disposable underwears, disposable diapers (adult and baby) including pull-on diapers and training pants, disposable panties for menstrual use, and disposable absorbent pads including sanitary napkins.

It is also known in the art that the exterior of disposable garments is covered with a flexible, liquid impervious and vapor pervious sheet to prevent any absorbed liquid from passing through the disposable garments and soiling adjacent articles such as clothing, bedding and the like. Such a liquid impervious sheet is generally referred to as a backsheet, and is often constructed from a fluid impervious sheet such as polyethylene film. While such backsheets do prevent liquid from passing through the disposable garments, the use of such a plastic film tends to provide an uncomfortable feeling to the wearer and/or user, since the touch of plastic film is very different from that of cloth garments.

To address this issue, recent disposable garments tend to employ a "cloth-like" backsheet wherein a nonwoven web is joined to the outer-facing surface (i.e., the garment-facing surface) of the plastic film to provide a "cloth-like" look and touch. While such a "cloth-like" backsheet can provide a soft and smooth touch, it tends not to have enough abrasion resistance sufficient to withstand a friction caused by the wearer's movement against adjacent articles such as clothing, bedding and the like. This results in undesirable fuzz occurrence, i.e., removal of at least some of the component fibers from the nonwoven web.

Based on the foregoing, there is a need for disposable garments which include a soft "cloth-like" backsheet that can reduce undesirable fuzz occurrence caused by friction against adjacent articles. There is also a need for a nonwoven composite laminate which can reduce undesirable fuzz occurrence caused by friction applied thereto.

SUMMARY

The present invention is directed to a nonwoven composite laminate. In one aspect, the nonwoven composite laminate includes: a first nonwoven layer including component fibers containing an ethylene-propylene random copolymer which contains from 7 mol% to 15 mol% of ethylene comonomer randomly distributed in the polymer backbone; and a second nonwoven insulation layer joined to the first nonwoven layer for preventing the smoothness oil from permeating therethrough.

The foregoing answers the need for disposable garments which include a soft "cloth-like" backsheet that can reduce undesirable fuzz occurrence caused by friction against adjacent articles. The foregoing also answers the need for a nonwoven composite laminate which can reduce undesirable fuzz occurrence caused by friction applied thereto.

These and other features, aspects, and advantages of the present invention will become evident to those skilled in the art from reading of the present disclosure.

BRIEF DESCRIPTION OF THE DRAWINGS

While the specification concludes with claims particularly pointing out and distinctly claiming the invention, it is believed that the invention will be better understood from the following description of preferred embodiments taken in conjunction with the accompanying drawings wherein like designations are used to designate substantially identical elements, and in which:

Fig. 1 is a perspective view of one preferred embodiment of the disposable pull-on diaper of the present invention in a typical configuration of use;

Fig. 2 is a simplified plan view of the embodiment shown in Fig. 1 in its flat uncontracted condition showing the body-facing side of the garment;

Fig. 3 is a cross-sectional view of a preferred embodiment taken along the section line 3-3 of Fig. 2;

Figs. 4 and 5 are schematic diagrams explaining measurement of the Surface Roughness of a sample nonwoven;

Figs. 6 and 7 are schematic diagrams explaining measurement of the surface friction of a sample nonwoven;

Fig. 8 shows the condition of the steel plate used for the Surface Roughness and friction measurements;

Fig. 9 shows the change in the coefficient of friction along the surface of a sample nonwoven; and

Fig. 10 shows the change in thickness along the surface of a sample nonwoven.

## DETAILED DESCRIPTION

Herein, "comprise", "include" and "contain" mean that other element(s) and step(s) which do not affect the end result can be added. These terms encompass the terms "consisting of" and "consisting essentially of".

Herein, "pull-on garment" refers to articles of wear which have a defined waist opening and a pair of leg openings and which are pulled onto the body of the wearer by inserting the legs into the leg openings and pulling the article up over the waist.

Herein, "disposable" describes garments which are not intended to be laundered or otherwise restored or reused as a garment (i.e., they are intended to be discarded after a single use and, preferably, to be recycled, composted or otherwise disposed of in an environmentally compatible manner).

Herein, "pull-on diaper" refers to pull-on garments generally worn by infants and other incontinent individuals to absorb and contain urine and feces. It should be understood, however, that the present invention is also applicable to other pull-on garments such as training pants, incontinent briefs, feminine hygiene garments or panties.

Herein, "nonwoven" may include any material which has been formed without the use of textile weaving processes which produce a structure of individual fibers interwoven in an identifiable manner. Methods of making suitable nonwovens includes a carded nonwoven process or a spunbonded nonwoven process.

Herein, "panel" denotes an area or element of the pull-on garment. While a panel is typically a distinct area or element, a panel may coincide (functionally correspond) somewhat with an adjacent panel.

Herein, "layer" does not necessarily limit the element to a single strata of material in that a layer may actually comprise laminates or combinations of sheets or webs of the requisite type of materials.

Herein, "joined" or "joining" encompasses configurations whereby an element is directly secured to another by affixing the element directly to the other element, and configurations whereby the element is indirectly secured to the other element by affixing the element to intermediate member(s) which in turn are affixed to the other element.

Herein, "uncontracted state" is used to describe states of pull-on garments in their unseamed (i.e., seams are removed), flat and relaxed condition wherein all elastic materials used are removed therefrom.

As indicated hereinbefore, the nonwoven composite laminate of the present invention includes a first nonwoven layer and a second nonwoven layer joined to the first nonwoven layer. The first nonwoven layer of the present invention includes component fibers which contain an ethylene-propylene random copolymer which contains from 7 mol% to 15 mol% of ethylene comonomer randomly distributed in the polymer backbone. The component fibers can have either a mono- (or single-) component fiber structure or a plural-component fiber structure. In a preferred embodiment, the component fibers have a bi-component fiber structure, more preferably a sheath/core structure. The sheath contains an ethylene-propylene random copolymer which contains from 7 mol% to 15 mol% of ethylene comonomer randomly distributed in the polymer backbone. The core of the bi-component fibers can be formed by any thermoplastic resin material known in the art. In a preferred embodiment, the material preferably used for the core of the bi-component fibers is a polypropylene. Preferably, the ratio by weight of the sheath to the core of the bi-component fibers is from 20:80 to 60:40. In a preferred embodiment, the ratio by weight of the sheath to the core of the bi-component fibers is about 40:60.

Preferably, from 8 mol% to 12 mol%, more preferably from 8.5 mol% to 10 mol% of ethylene comonomer is randomly distributed in the polymer backbone. In a preferred embodiment, about 8.5 mol% of ethylene comonomer is randomly distributed in the polymer backbone.

The ethylene-propylene random copolymer of the present invention has a PEP Ratio of from 50 mol% to 100 mol%. Herein, "PEP Ratio" refers to a mol% of the monomer sequence units of "propylene-ethylene-propylene" among the ethylene-centered triads which are contained in an ethylene-propylene random copolymer. Herein, "triad" refers to a monomer sequence unit including three monomers sequentially bonded in the polymer backbone of a polymer. The triad can be picked up or selected from any portion in the polymer backbone. Herein, "ethylene-centered triad" refers to a triad that contains an ethylene comonomer at least in the center position in the triad. Examples of the ethylene-centered triads include "propylene-ethylene-propylene", "ethylene-ethylene-propylene", and "ethylene-ethylene-ethylene".

Preferably, the ethylene-propylene random copolymer has a PEP Ratio of from 60 mol% to 80 mol%. In a preferred embodiment, the ethylene-propylene random copolymer has a PEP Ratio of about 70 mol%.

There are a variety of analytical methods suitable for determining the PEP Ratio in random copolymers. Any suitable method can be used. A preferred method is the $^{13}$C Nuclear Magnetic Resonance (NMR) spectroscopy method suggested by M. Kakugo et al. (Macromolecules 15, 1150-1152, (1982)). This method is explained in more detail in the Test Method Section.

By employing the first nonwoven layer in the nonwoven composite laminate of the present invention, the nonwoven composite laminate can provide enough abrasion resistance against a friction which may be generated by the wearer's (or user's) movement against adjacent materials. Consequently, the nonwoven composite laminate can reduce undesired fuzz occurrence caused by friction, while providing a soft "cloth-like" look and a soft/smooth touch.

The ethylene-propylene random copolymer contained in the component fibers can be produced by copolymerizing from 7 mol% to 15 mol% of ethylene comonomer with a propylene comonomer so that the ethylene comonomer can be randomly distributed in the polymer backbone of polypropylene.

The ethylene-propylene random copolymer is less crystalline and thus has a broader melting endotherm range than polypropylene itself. Preferably, the ethylene-propylene copolymer has a melting endotherm range at least between 80 °C and 165 °C, more preferably between 60 °C and 185 °C. The broad melting endotherm range allows for heat bonding of the component fibers over a wider range of nonwoven formation temperatures. Thus, the component fibers can be bonded stably through a nonwoven formation process, whereby the abrasion resistance against friction of the resulting nonwoven web can be improved.

In a preferred embodiment, the first nonwoven layer has a basis weight of less than 30 g/m², and comprises bi-component fibers having an average fiber denier of less than 2.5. Preferably, for products such as disposable diapers and the like, the nonwoven layer has a basis weight of from 5 g/m² to 23 g/m², more preferably from 10 g/m² to 18 g/m², and an average fiber denier of less than 2, and more preferably less than 1.7.

In a preferred embodiment, the first nonwoven layer includes at least 10% by weight of the component fibers of the ethylene-propylene random copolymer, and supplementary fibers. Herein, "supplementary fibers" are fibers which are different from the component fibers of the ethylene-propylene random copolymer in terms of materials used therein, fiber structure, physical properties or dimensions. The supplementary fibers can be any type of fibers known in the art, for example, synthetic fibers (e.g., polymeric fibers such as polyester, polypropylene, or polyethylene fibers), natural fibers (e.g., wood or cotton fibers), or a combination of natural and synthetic fibers. In a preferred embodiment, the supplementary fibers are mono-component fibers formed by a polypropylene. Preferably, the first nonwoven layer includes 30% by weight of the component fibers of the ethylene-propylene random copolymer and 70% of the supplementary fibers. More preferably, the first nonwoven layer includes more than 90% by weight of the component fibers of the ethylene-propylene random copolymer. When the first nonwoven layer includes supplementary fibers, the supplementary fibers are mixed or entangled with the component fibers of the ethylene-propylene random copolymer. In a preferred embodiment, the first nonwoven layer includes 100% by weight of the bi-component fibers (i.e., no supplementary fibers included).

In a preferred embodiment, the first nonwoven layer has a Surface Roughness of less than 4 $\mu$m, preferably less than 3.7 $\mu$m, and more preferably from 1.5 $\mu$m to 3.5 $\mu$m. The method for measuring the Surface Roughness of nonwoven webs or layers is explained in detail in the Test Method Section.

In a preferred embodiment, the first nonwoven layer has a Coefficient of Friction of less than 0.25, preferably less than 0.2 and more preferably from 0.1 to 0.2. The method for measuring the Coefficient of Friction of nonwoven webs or layers is explained in detail in the Test Method Section.

The second nonwoven layer may be formed by any nonwoven manufacture process known in the art such as a carding process, a spunbonding process or a meltblowing process. In a preferred embodiment, the second nonwoven layer is formed by a polypropylene based polymer. The second nonwoven layer of polypropylene based polymer may be any type of nonwoven web that is formed by a homo-polypropylene, a propylene-segmental polymer, or a polymer mixture or alloy with a polypropylene. In a preferred embodiment, the second nonwoven layer of polypropylene based polymer is a carded or spunbonded nonwoven web formed by component fibers of about 100% by weight of polypropylene.

In a preferred embodiment, the second nonwoven layer has a basis weight of less than 30 g/m², and comprises component fibers having an average fiber denier of less than 2.5. Preferably, for products such as disposable diaper and the like, the second nonwoven layer has a basis weight of from 5 g/m² to 23 g/m², more preferably from 10 g/m² to 18 g/m², and an average fiber denier of less than 2, and more preferably less than 1.7.

The second nonwoven layer may be joined to the first nonwoven layer by any suitable attachment means known in the art. For example, the second nonwoven layer may be secured to the first nonwoven layer by an adhesive or a heat/pressure bond. Herein, "heat/pressure bond" is either a physical or chemical bond formed by an application of appropriate heat and pressure between two different members. The bonding pattern can be in the form of a continuous plane, separate lines, spirals, spots, and the like. In a preferred embodiment, the second nonwoven layer is bonded to the first nonwoven layer by forming a heat/pressure bond between the first and second nonwoven layers. While such a heat/pressure bond can be preferably formed by an embossment formation process (which is described below), it can be formed by an independent process which is independent from the embossment formation process.

In a preferred embodiment, the nonwoven composite laminate has an embossment pattern formed by a number of discrete bonding spots or areas. Any embossment process known in the art can be used for forming such an embossment pattern in the first nonwoven layer. For example, a preferred embossment pattern is formed by feeding a nonwoven web through two bonding rolls which include a flat roll and an embossing roll which has a raised pattern such as spots or grids on the surface thereof. The bonding rolls are heated to a melting endotherm range of the ethylene-propylene random copolymer of the component fibers. The nonwoven web passes between the heated bonding rolls so that the nonwoven web can be compressed and heated by the bonding rolls in accordance with the pattern on the rolls, thereby forming an embossment pattern of discrete bonding spots or areas in the nonwoven web. The resulting nonwoven web is preferably used as the first nonwoven layer. As is well known in the art, the embossment holds the component fibers of the ethylene-propylene random copolymer together and imparts an integrity to the nonwoven web or layer by bonding the component fibers within the nonwoven web or layer.

Preferably, the embossment pattern on the embossing roll has an embossment area (or a bonded area) ratio of less than 20%. In a preferred embodiment, the embossment area ratio of the nonwoven web or layer is from 7% to 15%, and more preferably from 9% to 13%.

The first nonwoven layer includes component fibers which contain a smoothness oil therein. The second nonwoven layer works as an insulation layer for preventing the smoothness oil from permeating therethrough. Herein, "smoothness oil" refers to an oil material which works to improve surface texture of a nonwoven web or layer which is formed by the component fibers containing the oil material. A preferred smoothness oil includes a silicone oil which contains a silicone compound.

Since the second nonwoven layer works as an insulation layer, the smoothness oil in the first nonwoven layer is prevented from permeating through the insulation layer. As a result, it is possible to prevent the smoothness oil from affecting the adherence ability of the adhesive which is used to secure the nonwoven composite laminate to other element (s) of disposable articles.

Preferably, the second nonwoven layer which works as the isolation layer of the present invention has a basis weight of less than 30 g/m$^2$, and comprises component fibers having an average fiber denier of less than 2.5. Preferably, for products such as disposable diaper, the second nonwoven layer has a basis weight of from 5 g/m$^2$ to 23 g/m$^2$, more preferably from 10 g/m$^2$ to 18 g/m$^2$, and an average fiber denier of less than 2, and more preferably less than 1.7.

In a preferred embodiment, the basis weight ratio of the first nonwoven layer to the nonwoven composite laminate is from 10% to 90%, more preferably from 30% to 70%, and yet more preferably about 50%.

Preferred nonwoven composite laminates are available from Vliesstoffwerk Christian Heinrich Sandler GmbH & Co. KG, Schwarzenbach/Saale, Germany, under Code Nos. VP22/98/91 and VP22/98/101. These nonwoven composite laminates have the following properties. (The ethylene-propylene random copolymer which forms the sheath of NBF(P-2) fibers contains 8.5 mol% of ethylene comonomer, and has PEP Ratio of 70 mol%.) In these nonwoven composite laminates, the basis weight ratio of the first nonwoven layer to the nonwoven composite laminate is about 50%.

Table

| | Unit | Example 1 | Example 2 |
|---|---|---|---|
| Supplier Name and Code Nos. | | Sandler VP22/98/91 | Sandler VP22/98/101 |
| First Nonwoven Layer: (Specification for Component Fibers) | | Daiwabo NBF(P-2) 1.7d x 45mm without Silicone Oil | Daiwabo NBF(P-2) 1.7d x 45mm with Silicone Oil |
| Second Nonwoven Layer: (Specification for Component Fibers) | | FiberVisions Hy-Dry T 1.5d x 40mm | FiberVisions Hy-Dry T 1.5d x 40mm |
| Basis Weight | (g/m$^2$) | 27.9 | 27.9 |
| Stiffness/Softness -Coefficient of Friction -Surface Roughness (flat roll side) | (microns) | 0.20 3.90 | 0.21 3.73 |

The nonwoven composite laminate of the present invention can be applied to a variety of disposable articles in need of a soft "cloth-like" touch or feel while reducing undesirable fuzz occurrence caused by friction against adjacent articles. Preferred disposable articles include sweat bands, bandages, body wraps, wipes, disposable underwears, disposable garments including disposable diapers (adult and baby) including pull-on diapers and training pants, disposable panties for menstrual use, and disposable absorbent pads including sanitary napkins and incontinence devices. In a preferred embodiment, the present invention can be applied to a disposable garment. In the following, a disposable pull-on diaper will be described in detail as one preferred embodiment of the present invention.

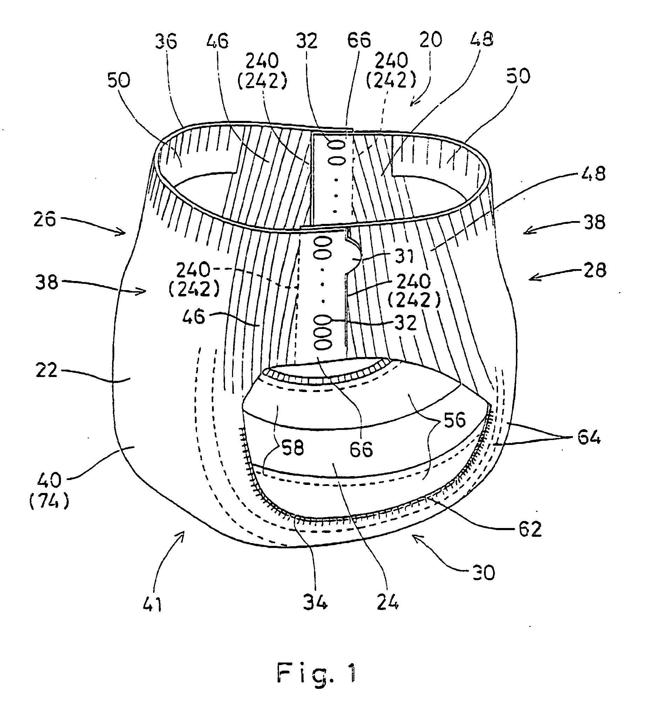

Referring to Fig. 1, the disposable pull-on diaper 20 has a front region 26; a back region 28 and a crotch region 30 between the front region 26 and the back region 28. A chassis 41 is disposed in the front, back and crotch regions 26, 28 and 30. The chassis 41 includes a liquid pervious topsheet 24, a liquid impervious backsheet 22 associated with the topsheet 24, and an absorbent core 25 (not shown in Fig. 1) disposed between the topsheet 24 and the backsheet 22.

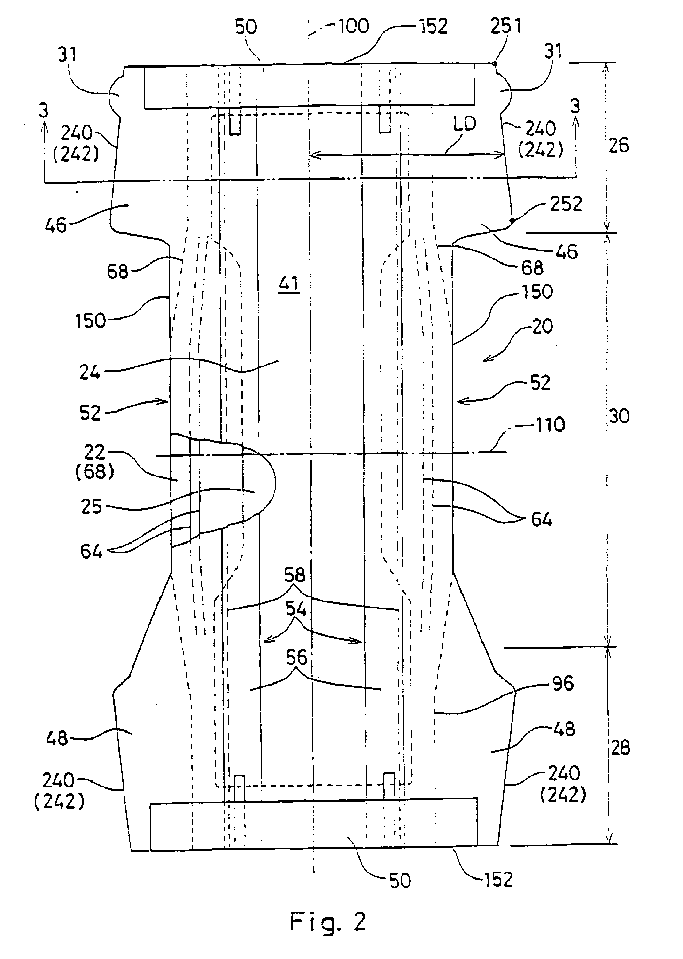

The disposable pull-on diaper 20 further includes a pair of front ear panels 46 each extending laterally outward from the corresponding sides of the chassis 41 in the front region 26, and a pair of extensible back ear panels 48 each extending laterally outward from the corresponding sides of the chassis 41 in the back region 28. Each of the ear panels 46 and 48 has an outermost edge 240 which forms an outermost edge line 242. At least one of the outermost edge lines 242 has a nonuniform lateral distance LD from the longitudinal center line 100 (not shown in Fig. 1 but in Fig. 2) in the uncontracted state of the garment 20. The pull-on diaper 20 further includes seams 32 each joining the front and back ear panels 46 and 48 along the corresponding edge lines 242 to form the two leg openings 34 and the waist opening 36.

In preferred embodiments, the pull-on diaper 20 includes a chassis layer 40 which generally determines the overall shape of the pull-on diaper 20. In the embodiment shown in Fig. 1, the chassis layer 40 is an outer cover nonwoven layer 74 which covers all of the outer-facing surface of the pull-on diaper 20 to provide the feel and appearance of a cloth garment. Preferably, the outer cover nonwoven layer 74 is a continuous sheet or web formed by the nonwoven composite laminate of the present invention. The continuous sheet (i.e., the outer cover nonwoven layer 74) defines the front region 26, the back region 28 and the crotch region 30 between the front region 26 and the back region 28. Each of the ear panels 46 and 48 includes a portion of the chassis layer 40. Preferred pull-on diapers which includes such a continuous sheet are disclosed in U.S. Patent No. 5,569,234 issued to Buell et al. on October 29, 1996. In a preferred embodiment, the continuous sheet is formed by one of the nonwoven composite laminates which are available from Vliesstoffwerk Christian Heinrich Sandler GmbH & Co. KG, Schwarzenbach/Saale, Germany, under Code Nos. VP22/98/91 and VP22/98/101.

In a preferred embodiment, at least one of, more preferably both of, the pairs of the ear panels 46 and 48 are elastically extensible in at least the lateral direction. In alternative embodiments, the ear panels 46 and 48 are elastically extensible both in the lateral and longitudinal directions. Herein, "extensible" refers to materials that are capable of extending in at least one direction to a certain degree without undue rupture. Herein, "elasticity" and "elastically extensible" refer to extensible materials that have the ability to return to approximately their original dimensions after the force that extended the material is removed. Herein, any material or element described as "extensible" may also be elastically extensible unless otherwise provided. The extensible ear panels 46 and 48 provide a more comfortable and contouring fit by initially conformably fitting the pull-on diaper to the wearer and sustaining this fit throughout the time of wear well past when the pull-on diaper has been loaded with exudates since the ear panels 46 and/or 48 allow the sides of the pull-on diaper to expand and contract.

The ear panels 46 and 48 may be formed by unitary elements of the pull-on diaper 20 (i.e., they are not separately manipulative elements secured to the pull-on diaper 20, but rather are formed from and are extensions of one or more of the various layers of the pull-on diaper). In a preferred embodiment, the ear panels 46 and 48 include at least one unitary element or a continuous sheet (e.g. the chassis layer 40) that forms a part of the chassis 41 and continuously extends into the ear panels 46 and 48. Alternatively, the ear panels 46 and 48 may only include discrete members (not shown in Figs.) which do not have any unitary element that also forms a part of the chassis 41. Such an ear panel structure may be formed by joining the discrete members to the corresponding sides of the chassis 41.

In a preferred embodiment, the pull-on diaper 20 further includes seam panels 66 each extending laterally outward from each of the ear panels 46 and 48; and tear open tabs 31 each extending laterally outward from the seam panel 66. In a preferred embodiment, each of the seam panels 66 is an extension of the corresponding ear panels 46 and 48, or at least one of the component elements used therein (e.g., the chassis layer 40), or any other combination of the elements. More preferably, each of the tear open tabs 31 is also an extension of the corresponding seam panel 66 or at least one of its component elements used therein (e.g., the chassis layer 40), or any other combination of its elements.

In a preferred embodiment, the corresponding edge portions of the ear panels 46 and 48 are joined through the seam panels 66 in an overlapping manner to make an overlapped seam structure as shown in Fig. 1. Alternatively, the front and ear panels 46 and 48 can be seamed in a butt seam manner (not shown in Figs.). The bonding of the seams 32 can be performed by any suitable means known in the art appropriate for the specific materials employed in the chassis 41 and/or the ear panels 46 and 48. Thus, sonic sealing, heat sealing, pressure bonding, adhesive or cohesive bonding, sewing or autogeneous bonding may be appropriate techniques. Preferably, the seam panels 66 are joined by a predetermined pattern of heat/pressure or ultrasonic welds which withstands the forces and stresses generated on the garment 20 during wear.

A continuous belt 38 is formed by the ear panels 46 and 48, and a part of the chassis 41 about the waist opening 36 as shown in Fig. 1. Preferably, elasticized waist bands 50 are provided in both the front region 26 and the back region 28. The continuous belt 38 acts to dynamically create fitment forces in the pull-on diaper 20 when positioned on the wearer, to maintain the pull-on diaper 20 on the wearer even when loaded with body exudates thus keeping the absorbent core 25 (not shown in Fig. 1) in close proximity to the wearer, and to distribute the forces dynamically generated during wear about the waist thereby providing supplemental support for the absorbent core 25 without binding or bunching the absorbent core 25.

Fig. 2 is a partially cut-away plan view of the pull-on diaper 20 of Fig. 1 in its uncontracted state (except in the ear panels 46 and 48 which are left in their relaxed condition) with the topsheet 24 facing the viewer, prior to the ear panels 46 and 48 being joined together by the seams 32. The pull-on diaper 20 has the front region 26, the back region 28 opposed to the front region 26, the crotch region 30 positioned between the front region 26 and the back region 28, and a periphery which is defined by the outer perimeter or edges of the pull-on diaper 20 in which the side edges are designated 150 and 240, and the end edges or waist edges are designated 152. The topsheet 24 has the body-facing surface of the pull-on diaper 20 which is positioned adjacent to the wearer's body during use. The backsheet 22 has the outer-facing surface of the pull-on diaper 20 which is positioned away from the wearer's body. The pull-on diaper 20 includes the chassis 41 including the liquid pervious topsheet 24, the liquid impervious backsheet 22 associated with the topsheet 24, and the absorbent core 25 positioned between the topsheet 24 and the backsheet 22. The garment 20 further includes the front and back ear panels 46 and 48 extending laterally outward from the chassis 41, the elasticized leg cuffs 52, and the elasticized waistbands 50. The topsheet 24 and the backsheet 22 have length and width dimensions generally larger than those of the absorbent core 25. The topsheet 24 and the backsheet 22 extend beyond the edges of the absorbent core 25 to thereby form the side edges 150 and the waist edges 152 of the garment 20. The liquid impervious backsheet 22 preferably includes a liquid impervious plastic film 68.

The pull-on diaper 20 also has two centerlines, a longitudinal centerline 100 and a transverse centerline 110. Herein, "longitudinal" refers to a line, axis, or direction in the plane of the pull-on diaper 20 that is generally aligned with (e.g. approximately parallel with) a vertical plane which bisects a standing wearer into left and right halves when the pull-on diaper 20 is worn. Herein, "transverse" and "lateral" are interchangeable and refer to a line, axis or direction which lies within the plane of the pull-on diaper that is generally perpendicular to the longitudinal direction (which divides the wearer into front and back body halves). The pull-on diaper 20 and component materials thereof also have a body-facing surface which faces the skin of wearer in use and an outer-facing surface which is the opposite surface to the body-facing surface.

While the topsheet 24, the backsheet 22, and the absorbent core 25 may be assembled in a variety of well known configurations, exemplary chassis configurations are described generally in U.S. Patent 3,860,003 entitled "Contractible Side Portions for Disposable Diaper" which issued to Kenneth B. Buell on January 14, 1975; and U.S. Patent 5,151,092 entitled "Absorbent Article With Dynamic Elastic Waist Feature Having A Predisposed Resilient Flexural Hinge" which issued to Kenneth B. Buell et al., on September 29, 1992.

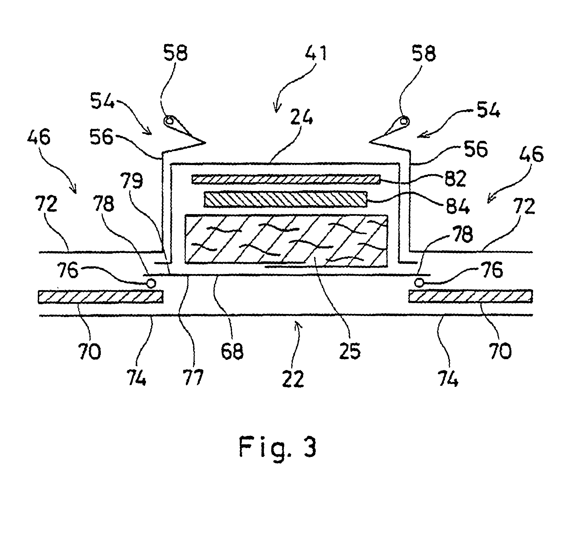

Fig. 3 is a cross-sectional view of a preferred embodiment taken along the section line 3-3 of Fig. 2. The pull-on diaper 20 includes the chassis 41 including the liquid pervious topsheet 24, the liquid impervious backsheet 22 associated with the topsheet 24, and the absorbent core 25 positioned between the topsheet 24 and the backsheet 22. The pull-on diaper 20 further includes the front ear panels 46 each extending laterally outward from the chassis 41, and an inner barrier cuffs 54. Although Fig. 3 depicts only the structure of the front ear panel 46 and the chassis 41 in the front region 26, preferably a similar structure is also provided in the back region 28. In a preferred embodiment, each of the front ear panels 46 is formed by a lamination of an extended part 72 of the barrier flap 56, an elastic member 70 and the outer cover nonwoven layer 74. The elastic member 70 includes a plane elastomeric material layer (not shown in Fig. 3). Herein, "plane elastomeric material" refers to elastomeric materials which continuously extend in two dimensional directions. Preferred plane elastomeric materials include a scrim, a perforated (or apertures formed) film, an elastomeric woven or nonwoven and an elastomeric foam. In a preferred embodiment, the plane elastomeric material layer includes at least a portion that has a nonuniform lateral width.

The absorbent core 25 can be any absorbent member which is generally compressible, conformable, non-irritating to the wearer's skin, and capable of absorbing and retaining liquids such as urine and other certain body exudates. The absorbent core 25 may be manufactured in a wide variety of sizes and shapes (e.g., rectangular, hourglass, "T"-shaped, asymmetric, etc.) and from a wide variety of liquid-absorbent materials commonly used in disposable pull-on garments and other absorbent articles such as comminuted wood pulp which is generally referred to as airfelt. Examples of other suitable absorbent materials include creped cellulose wadding; meltblown polymers including coform; chemically stiffened, modified or cross-linked cellulosic fibers; tissue including tissue wraps and tissue laminates; absorbent foams; absorbent sponges; superabsorbent polymers and absorbent gelling materials.

The configuration and construction of the absorbent core 25 may vary (e.g., the absorbent core 25 may have varying caliper zones, a hydrophilic gradient, a superabsorbent gradient, or lower average density and lower average basis weight acquisition zones; or may include one or more layers or structures). Further, the size and absorbent capacity of the absorbent core 25 may also be varied to accommodate wearers ranging from infants through adults. However, the total absorbent capacity of the absorbent core 25 should be compatible with the design loading and the intended use of the garment 20.

A preferred embodiment of the garment 20 has an asymmetric, modified hourglass-shaped absorbent core 25 having ears in the front and back waist regions 26 and 28. Other exemplary absorbent structures for use as the absorbent core 25 that have achieved wide acceptance and commercial success are described in U.S. Patent No. 4,610,678 entitled "High-Density Absorbent Structures" issued to Weisman et al. on September 9, 1986; U.S. Patent No. 4,673,402 entitled "Absorbent Articles With Dual-Layered Cores" issued to Weisman et al. on June 16, 1987; U.S. Patent No. 4,888,231 entitled "Absorbent Core Having A Dusting Layer" issued to Angstadt on December 19, 1989; and U.S. Patent No. 4,834,735, entitled "High Density Absorbent Members Having Lower Density and Lower Basis Weight Acquisition Zones", issued to Alemany et al. on May 30, 1989.

The chassis 41 may further include an acquisition/distribution core 84 of chemically stiffened fibers positioned over the absorbent core 25, thereby forming a dual core system. In a preferred embodiment, the fibers are hydrophilic chemically stiffened cellulosic fibers. Herein, "chemically stiffened fibers" means any fibers which have been stiffened by chemical means to increase stiffness of the fibers under both dry and aqueous conditions. Such means include the addition of chemical stiffening agents which, for example, coat and/or impregnate the fibers. Such means also include the stiffening of the fibers by altering the chemical structure of the fibers themselves, e.g., by cross-linking polymer chains.

The fibers utilized in the acquisition/distribution core 84 can also be stiffened by means of chemical reaction. For example, crosslinking agents can be applied to the fibers which, subsequent to application, are caused to chemically form intrafiber crosslink bonds. These crosslink bonds can increase stiffness of the fibers. Whereas the utilization of intrafiber crosslink bonds to chemically stiffen the fibers is preferred, it is not meant to exclude other types of reactions for chemical stiffening of the fibers.

In the more preferred stiffened fibers, chemical processing includes intrafiber crosslinking with crosslinking agents while such fibers are in a relatively dehydrated, defibrated (i.e. individualized), twisted, curled condition. Suitable chemical stiffening agents include monomeric crosslinking agents including, but not limited to, $C_2$-$C_8$ dialdehydes and $C_2$-$C_8$ monoaldehydes having an acid functionality can be employed to form the cosslinking solution. These compounds are capable of reacting with at least two hydroxyl groups in a single cellulose chain or on proximately located cellulose chains in a single fiber. Such crosslinking agents contemplated for use in preparing the stiffened cellulose fibers include, but are not limited to, glutaraldehyde, glyoxal, formaldehyde, and glyoxylic acid. Other suitable stiffening agents are polycarboxylates, such as citric acid. The polycarboxylic stiffening agents and a process for making stiffened fibers from them are described in U.S. Patent No. 5,190,563, entitled "Process for Preparing Individualized, Polycarboxylic Acid crosslinked Fibers" issued to Herron, on March 2, 1993. The effect of crosslinking under these conditions is to form fibers which are stiffened and which tend to retain their twisted, curled configuration during use in the absorbent articles herein. Such fibers, and processes for making them are cited in the above patents.

Preferred dual core systems are disclosed in U.S. Patent No. 5,234,423, entitled "Absorbent Article With Elastic Waist Feature and Enhanced Absorbency" issued to Alemany et al., on August 10, 1993; and in U.S. Patent No. 5,147,345, entitled "High Efficiency Absorbent Articles For Incontinence Management" issued to Young, LaVon and Taylor on September 15, 1992. In a preferred embodiment, the acquisition/distribution core 84 includes chemically treated stiffened cellulosic fiber material, available from Weyerhaeuser Co. (U.S.A.) under the trade designation of "CMC". Preferably, the acquisition/distribution core 84 has a basis weight of from 40 g/m$^2$ to 400 g/m$^2$, more preferably from 75 g/m$^2$ to 300 g/m$^2$.

More preferably, the chassis 22 further includes an acquisition/distribution layer 82 between the topsheet 24 and the acquisition/distribution core 84 as shown in Fig. 3. The acquisition/distribution layer 82 is provided to help reduce the tendency for surface wetness of the topsheet 24. The acquisition/distribution layer 82 preferably includes carded, resin bonded hiloft nonwoven materials such as, for example, available as Code No. FT-6860 from Polymer Group, Inc., North America (Landisiville, New Jersey, U.S.A.), which is made of polyethylene telephthalate fibers of 6 dtex, and has a basis weight of about 43 g/m$^2$. A preferable example for the acquisition/distribution layer 82 and the acquisition/distribution core 84 is disclosed in EP 0797968A1 (Kurt et al.) published on October 1, 1997.

The topsheet 24 is preferably compliant, soft feeling, and non-irritating to the wearer's skin. Further, the topsheet 24 is liquid pervious permitting liquids (e.g., urine) to readily penetrate through its thickness. A suitable topsheet 24 may be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be included of natural fibers (e.g., wood or cotton fibers), synthetic fibers (e.g., polymeric fibers such as polyester, polypropylene, or polyethylene fibers) or from a combination of natural and synthetic fibers. The topsheet 24 is preferably made of a hydrophobic material to isolate the wearer's skin from liquids which have passed through the topsheet 24 and are contained in the absorbent core 25 (i.e., to prevent rewet). In a preferred embodiment, the topsheet 24 is formed by the nonwoven composite laminate of the present invention. If the topsheet 24 is made of a hydrophobic material, at least the upper surface of the topsheet 24 is treated to be hydrophilic so that liquids will transfer through the topsheet more rapidly. This diminishes the likelihood that body exudates will flow off the topsheet 24 rather than being drawn through the topsheet 24 and being absorbed by the absorbent core 25. The topsheet 24 can be rendered hydrophilic by treating it with a hydrophilic finishing oil or a surfactant. Suitable methods for the treatment for the topsheet 24 include spraying the topsheet 24 material with the surfactant and immersing the material into the surfactant. A more detailed discussion of such a treatment and hydrophilicity is contained in U.S. Patent No. 4,988,344 entitled "Absorbent Articles with Multiple Layer Absorbent Layers" issued to Reising, et al. on January 29, 1991 and U.S. Patent No. 4,988,345 entitled "Absorbent Articles with Rapid Acquiring Absorbent Cores" issued to Reising on January 29,1991. Alternatively, the topsheet 24 may be a carded nonwoven material which is formed by hydrophilic component fibers.

In a preferred embodiment, the topsheet 24 is a nonwoven web that can provide reduced tendency for surface wetness; and consequently facilitate maintaining urine absorbed by the core 25 away from the user's skin, after wetting. One of the preferred topsheet materials is a thermobonded carded web which is available as Code No. P-8 from Fiberweb North America, Inc. (Simpsonville, South Carolina, U.S.A.). Another preferred topsheet material is available as Code No. S-2355 from Havix Co., Japan. This material is a bi-layer composite material, and made of two kinds of synthetic surfactant treated bi-component fibers by using carding and air-through technologies. Yet another preferred topsheet material is a thermobonded carded web which is available as Code No. Profleece Style 040018007 from Amoco Fabrics, Inc. (Gronau, Germany).

Another preferred topsheet 24 includes an apertured formed film. Apertured formed films are preferred for the topsheet 24 because they are pervious to body exudates and yet non-absorbent and have a reduced tendency to allow liquids to pass back through and rewet the wearer's skin. Thus, the surface of the formed film which is in contact with the body remains dry, thereby reducing body soiling and creating a more comfortable feel for the wearer. Suitable formed films are described in U.S. Patent No. 3,929,135, entitled "Absorptive Structures Having Tapered Capillaries", issued to Thompson on December 30, 1975; U.S. Patent No. 4,324,246 entitled "Disposable Absorbent Article Having A Stain Resistant Topsheet", issued to Mullane, et al. on April 13, 1982; U.S. Patent No. 4,342;314 entitled "Resilient Plastic Web Exhibiting Fiber-Like Properties", issued to Radel. et al. on August 3, 1982; U.S. Patent No. 4,463,045 entitled "Macroscopically Expanded Three-Dimensional Plastic Web Exhibiting Non-Glossy Visible Surface and Cloth-Like Tactile Impression", issued to Ahr et al. on July 31, 1984; and U.S. 5,006,394 "Multilayer Polymeric Film" issued to Baird on April 9, 1991.

In a preferred embodiment, the backsheet 22 includes the liquid impervious film 68. Preferably, the liquid impervious film 68 longitudinally extends in the front, back and crotch regions 26, 28 and 30 as shown in Fig. 2. More preferably, the liquid impervious film 68 does not laterally extend into the at least one of the ear panels 46 or 48. Referring again to Fig. 3, the liquid impervious film 68 has a body-facing surface 79 and an outer-facing surface 77 opposing the body-facing surface 79. The liquid impervious film 68 is impervious to liquids (e.g., urine) and is preferably manufactured from a thin plastic film. However, more preferably the plastic film permits vapors to escape from the garment 20. In a preferred embodiment, a microporous polyethylene film is used for the liquid impervious film 68. A suitable microporous polyethylene film is manufactured by Mitsui Chemicals, Inc., Tokyo, Japan and marketed in the trade as PG-P. In a preferred embodiment, a disposable tape (not shown in Figs.) is additionally joined to the outer surface of the backsheet 22 to provide a convenient disposal after soiling. A preferred disposal tape (or device) for pull-on garments is disclosed in International Publication No. WO 94/09736 (Rollag et al.) published on May 11, 1994.

A suitable material for the liquid impervious film 68 is a thermoplastic film having a basis weight of from 10 $g/m^2$ to 45 $g/m^2$. Preferably, the thermoplastic film has a basis weight of from 25 $g/m^2$ to 40 $g/m^2$. In a preferred embodiment, the thermoplastic film has a basis weight of about 35 $g/m^2$.

The backsheet 22 further includes the outer cover nonwoven layer 74 (i.e., the chassis layer 40) which is joined with the outer-facing surface 77 of the liquid impervious film 68 to form a laminate. Preferably, the outer cover nonwoven layer 74 is formed by the nonwoven composite laminate of the present invention. In a preferred embodiment, the nonwoven composite laminate is employed in the pull-on diaper 20 such that the first nonwoven layer forms at least part of the garment-facing surface of the diaper 20. The outer cover nonwoven layer 74 preferably covers substantially all of the outer-facing surface of the pull-on diaper 20 to provide a complete feel and appearance of a cloth garment. The outer cover nonwoven layer 74 may be joined to the liquid impervious film 68 by any suitable attachment means known in the art. For example, the outer cover nonwoven layer 74 may be secured to the liquid impervious film 68 by a uniform continuous layer of adhesive, a patterned layer of adhesive, or an array of separate lines, spirals, or spots of adhesive. Suitable adhesives include a hotmelt adhesive obtainable from Nitta Findley Co., Ltd., Osaka, Japan as H-2128, and a hotmelt adhesive obtainable from H.B. Fuller Japan Co., Ltd., Osaka, Japan as JM-6064.

In a preferred embodiment, the outer cover nonwoven layer 74 is formed by one of the nonwoven composite laminates which are available from Vliesstoffwerk Christian Heinrich Sandler GmbH & Co. KG, Schwarzenbach/Saale, Germany, under Code Nos. VP22/98/91 and VP22/98/101.

Preferably, the component fibers of the first nonwoven layer in the outer cover nonwoven layer 74 contain a smoothness oil therein so that it can improve surface texture of the outer cover nonwoven layer 74. In the meantime, the second nonwoven layer in the outer cover nonwoven layer 74 works as the insulation layer of the present invention, the smoothness oil in the first nonwoven layer is prevented from permeating through the insulation layer. As a result, it is possible to prevent the smoothness oil from affecting the adherence ability of the adhesive which is often used to secure the outer cover nonwoven layer 74 to the liquid impervious film 68.

The backsheet 22 is positioned adjacent the outer-facing surface of the absorbent core 25 and is preferably joined thereto by any suitable attachment means known in the art. Specifically, the body-facing surface 79 of the liquid impervious film 68 may be secured to the absorbent core 25 by a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minnesota, U.S.A., and marketed as HL-1358J. An example of a suitable attachment means including an open pattern network of filaments of adhesive is disclosed in U.S. Patent No. 4,573,986 entitled "Disposable Waste-Containment Garment", which issued to Minetola et al. on March 4, 1986. Another suitable attachment means including several lines of adhesive filaments swirled into a spiral pattern is illustrated by the apparatus and methods shown in U.S. Patent No. 3,911,173 issued to Sprague, Jr. on October 7, 1975; U.S. Patent No. 4,785,996 issued to Ziecker, et al. on November 22, 1978; and U.S. Patent No. 4,842,666 issued to Werenicz on June 27, 1989. Alternatively, the attachment means may include heat bonds, pressure bonds, ultrasonic bonds, dynamic mechanical bonds, or any other suitable attachment means or combinations of these attachment means as are known in the art.

In an alternative embodiment, the absorbent core 25 is not joined to the backsheet 22, and/or the topsheet 24 in order to provide greater extensibility in the front region 26 and the back region 28.

The pull-on diaper 20 preferably further includes elasticized leg cuffs 52 for providing improved containment of liquids and other body exudates. The elasticized leg cuffs 52 may include several different embodiments for reducing the leakage of body exudates in the leg regions. (The leg cuffs can be and are sometimes also referred to as leg bands, side flaps, barrier cuffs, elastic cuffs or gasketing cuffs.) U.S. Patent 3,860,003 entitled "Contractable Side Portions for Disposable Diaper" issued to Buell on January 14, 1975, describes a disposable diaper which provides a contractible leg opening having a side flap and one or more elastic members to provide an elasticized leg cuff. U.S. Patent 4,909,803 entitled "Disposable Absorbent Article Having Elasticized Flaps" issued to Aziz et al. on March 20, 1990, describes a disposable diaper having "stand-up" elasticized flaps (barrier cuffs) to improve the containment of the leg regions. U.S. Patent 4,695,278 entitled "Absorbent Article Having Dual Cuffs" issued to Lawson on September 22, 1987; and U.S. Patent 4,795,454 entitled "Absorbent Article Having Leakage-Resistant Dual Cuffs" issued to Dragoo on January 3, 1989, describe disposable diapers having dual cuffs including a gasketing cuff and a barrier cuff. U.S. Patent 4,704,115 entitled "Disposable Waist Containment Garment" issued to Buell on November 3, 1987, discloses a disposable diaper or incontinence garment having side-edge-leakage-guard gutters configured to contain free liquids within the garment. In a preferred embodiment, such barrier cuffs or "stand-up" elasticized flaps are formed by the nonwoven composite laminate of the present invention.

While each elasticized leg cuff 52 may be configured so as to be similar to any of the leg bands, side flaps, barrier cuffs, or elastic cuffs described above, it is preferred that the elasticized leg cuff 52 includes an elastic gasketing cuff 62 with one or more elastic strands 64 as shown in Fig. 2, which is described in the above-referred U.S. Patent Nos. 4,695,278 and 4,795,454. It is also preferred that each elasticized leg cuff 52 further includes inner barrier cuffs 54 each including a barrier flap 56 and a spacing means 58 which are described in the above-referenced U.S. Patent No. 4,909,803.

The pull-on diaper 20 preferably further includes an elasticized waistband 50 that provides improved fit and containment. The elasticized waistband 50 is that portion or zone of the pull-on diaper 20 which is intended to elastically expand and contract to dynamically fit the wearer's waist. The elasticized waistband 50 preferably extends longitudinally outwardly from the waist edge of the pull-on diaper 20 toward the waist edge of the absorbent core 25. Preferably, the pull-on diaper 20 has two elasticized waistbands 50, one positioned in the back region 28 and one positioned in the front region 26, although other pull-on garment embodiments can be constructed with a single elasticized waistband. The elasticized waistband 50 may be constructed in a number of different configurations including those described in U.S. Patent 4,515,595 entitled "Disposable Diapers with Elastically Contractible Waistbands" issued to Kievit et al. on May 7, 1985 and the above referenced U.S. Patent 5,151,092 issued to Buell.

The waistbands 50 may include materials that have been "prestrained" or "mechanically prestrained" (i.e., subjected to some degree of localized pattern mechanical stretching to permanently elongate the material). In a preferred embodiment, the nonwoven composite laminate of the present invention can be used in one of the component materials used in the waistbands 50. Alternatively, the materials may be prestrained by directing the material through an incremental mechanical stretching system as described in U.S. Patent No. 5,330,458 entitled "Absorbent Article With Elastic Feature Having A Portion Mechanically Prestrained" issued to Buell et al., on July 19, 1994. The materials are then allowed to return to their substantially untensioned condition, thus forming a "zero strain" stretch laminate that is extensible, at least up to the point of initial stretching. Examples of "zero strain" laminate are described hereinafter. In a preferred embodiment, the elasticized waistband 50 includes the nonwoven composite laminate of the present invention.

At least one pair of the ear panels 46 and 48 includes the elastic member 70 as shown in Fig. 3. In one embodiment of the present invention, at least one pair of the ear panels 46 and 48 includes the nonwoven composite laminate of the present invention. The elastic member 70 of the front ear panels 46 includes an elastomeric material layer (not shown in Fig. 3) which preferably extends laterally outward from the chassis 41 to provide good fitness by generating the optimal retention (or sustained) force at the waist and side areas of the wearer. Preferably, the elastomeric material layer is extensible in at least one direction, preferably in the lateral direction to generate a retention (or sustained) force that is optimal to prevent the pull-on diaper 20 from drooping, sagging, or sliding down from its position on the torso without causing the red marking on the skin of the wearer. In a preferred embodiment, each of the ear panels 46 and 48 includes the elastomeric material layer.

The elastic member 70 is operatively joined to the outer cover nonwoven layer 74 and preferably the nonwoven webs 72 in the ear panels 46 and 48 to form a laminate. In a preferred embodiment, the elastic member 70 is operatively joined to the nonwoven webs 72 and 74 by securing them to at least one, preferably both of the nonwoven webs 72 and 74 while in a substantially untensioned (zero strain) condition.

The elastic member 70 can be operatively joined to the nonwoven webs 72 and 74, by using either an intermittent bonding configuration or a substantially continuous bonding configuration. Herein, "intermittently" bonded laminate web means a laminate web wherein the plies are initially bonded to one another at discrete spaced apart points or a laminate web wherein the plies are substantially unbonded to one another at discrete spaced apart areas. Conversely, a "substantially continuously" bonded laminate web means a laminate web wherein the plies are initially bonded substantially continuously to one another throughout the areas of interface. It is preferred that the stretch laminate be bonded over all or a significant portion of the stretch laminate so that the inelastic webs (i.e., the nonwoven webs 72 and 74) elongate or draw without causing rupture, and the layers of the stretch laminates are preferably bonded in a configuration that maintains all of the layers of the stretch laminate in relatively close adherence to one another after the incremental mechanical stretching operation. Consequently, the elastic panel members and the other plies of the stretch laminate are preferably substantially continuously bonded together using an adhesive. In a particularly preferred embodiment, the adhesive selected is applied with a control coat spray pattern at a basis weight of about 7.0 grams/m$^2$. The adhesive pattern width is about 6.0 cm. The adhesive is preferably an adhesive such as is available from Nitta Findley Co., Ltd., Osaka, Japan, under the designation H2085F. Alternatively, the elastic panel member and any other components of the stretch laminates may be intermittently or continuously bonded to one another using heat bonding, pressure bonding, ultrasonic bonding, dynamic mechanical bonding, or any other method as is known in the art.

After the elastic member 70 is operatively joined to the nonwoven webs 72 and 74, at least a portion of the resultant composite stretch laminate is then subjected to mechanical stretching sufficient to permanently elongate the non-elastic components which are, for example, the nonwoven webs 72 and 74. The composite stretch laminate is then allowed to return to its substantially untensioned condition. At least one pair of, preferably both of the ear panels 46 and 48 is thus formed into "zero strain" stretch laminates. This configuration allows the ear panels 46 and 48 to be elastically extensible in at least the lateral direction.

A "zero strain" stretch laminate is one type of nonwoven composite laminate which is preferably used for such disposable products. For example, methods for making "zero strain" stretch laminate webs are disclosed in U.S. Patent No. 5,167,897 issued to Weber et al. on December 1, 1992; U.S. Patent No. 5,156,793 issued to Buell et al. on October 20, 1990; and U.S. Patent No. 5,143,679 issued to Weber et al. on September 1, 1992. In a manufacturing process for such "zero strain" stretch laminate, an elastomeric material is operatively joined to at least one non-elastic component material in a substantially untensioned (zero strain) condition. At least a portion of the resultant non-elastic composite stretch laminate is then subjected to mechanical stretching sufficient to permanently elongate the non-elastic component material. The composite stretch laminate is then allowed to return to its substantially untensioned condition. Thus, the nonwoven composite laminate is formed into a "zero strain" stretch laminate. Herein, "zero strain" stretch laminate refers to a laminate comprised of at least two plies of material which are secured to one another along at least a portion of their coextensive surfaces while in a substantially untensioned ("zero strain") condition; one of the plies comprising a material which is stretchable and elastomeric (i.e., will return substantially to its untensioned dimensions after an applied tensile force has been released) and a second ply which is elongatable (but not necessarily elastomeric) so that upon stretching the second ply will be, at least to a degree, permanently elongated so that upon release of the applied tensile forces, it will not fully return to its original undeformed configuration. The resulting stretch laminate is thereby rendered elastically extensible, at least up to the point of initial stretching, in the direction of initial stretching.

The elastic member 70 is preferably joined to, more preferably directly secured to the respective edges 78 of the liquid impervious film (i.e., the liquid impervious film 68) through an adhesive 76 as shown in Fig. 3. In a preferred embodiment, while liquid impervious film 68 longitudinally extends in the front, back and crotch regions 26, 28 and 30, it does not laterally extend into at least one of, preferably each of the extensible ear panels 46 and 48. In a more preferred embodiment, the elastic member 70 is joined to the respective edges 78 of the liquid impervious film 68 at the outer-facing surface 77 as shown in Fig. 3. In an alternative embodiment, the elastic member 70 may be joined to the respective edges 78 of the liquid impervious film 68 at the body-facing surface 79. Preferably, the adhesive 76 is applied in a spiral glue pattern. In a preferred embodiment, the adhesive 76 is a flexible adhesive with an amorphous and crystallizing component. Such a preferred adhesive is made by Nitta Findley Co., Ltd., Osaka, Japan, under the designation H2085F. Alternatively, the elastic member 70 may be joined to the respective edges 78 of the liquid impervious film 68 by any other bonding means known in the art which include heat bonds, pressure bonds, ultrasonic bonds, dynamic mechanical bonds, or combinations of these attachment means.

## TEST METHODS

### 1. PEP Ratio

A sample of random copolymer is dissolved in a solvent of 1,2-dichlorobenzene to prepare 5% weight per volume of solution. The sample can be any form of the material to be analyzed, for example, a chip of resin, fibers, or a piece of nonwoven. Benzene-d6 is added to the solution for capturing a lock signal, and tetramethylsilane is also added as an internal chemical shift reference (e.g., 0 ppm). The solution is analyzed by a Nuclear Magnetic Resonance (NMR) Spectrometer to obtain $^{13}$C NMR spectra. A preferred NMR Spectrometer is commercially available from JEOL Co. Ltd., Tokyo, Japan, under Trade Name "Lambda 500". The analysis is preferably conducted under the following conditions:

Frequency: 125,778 MHz
Measurement Temperature: 100°C
Number of Transients Accumulated: 500 - 1000

### 2. Surface Roughness and Coefficient of Friction

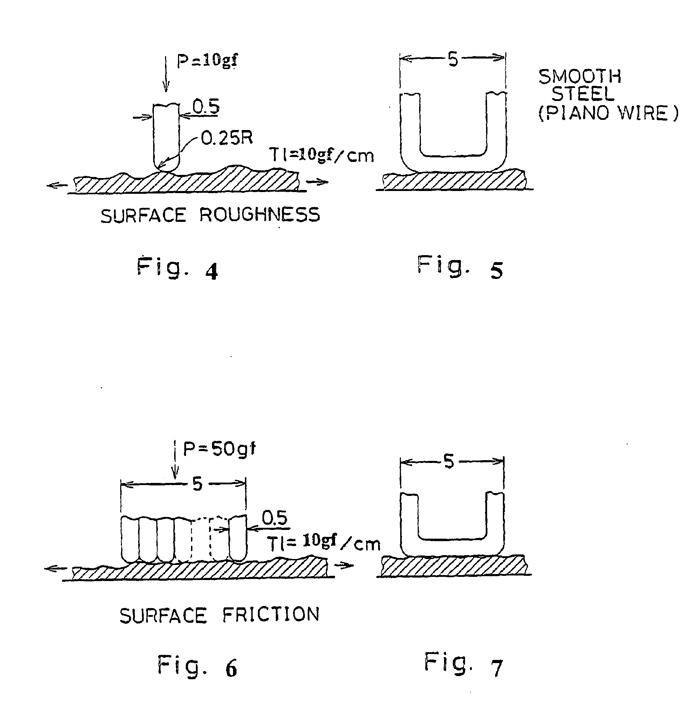

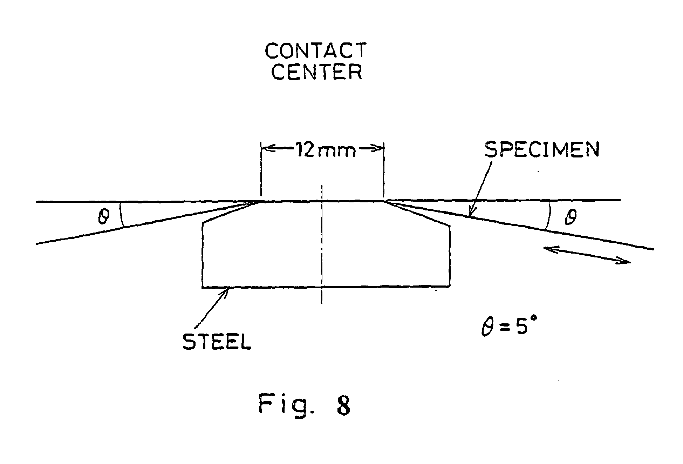

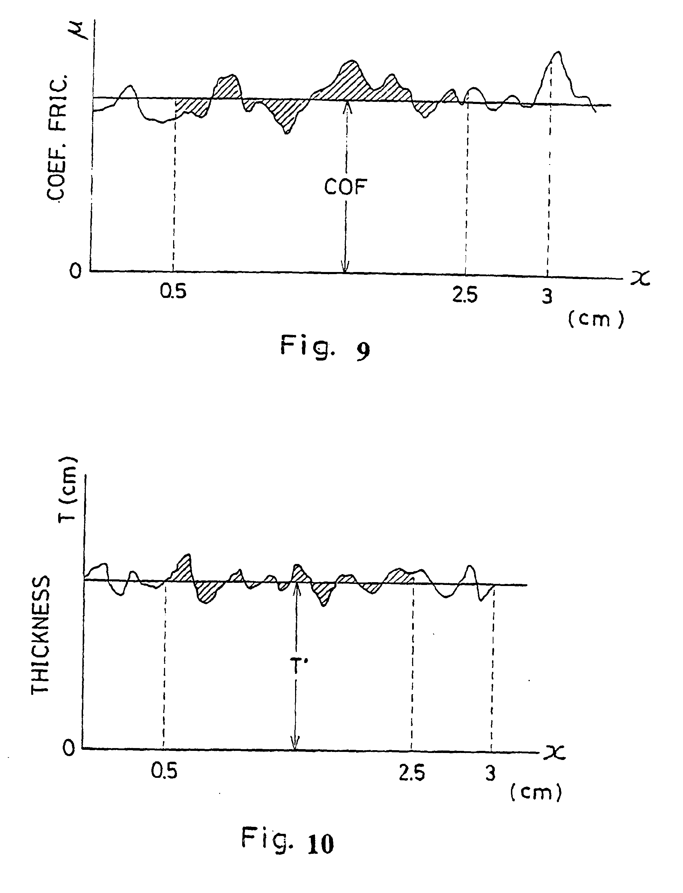

To measure the Surface Roughness of the specimen (i.e., a nonwoven layer), a pianowire is prepared and bent as shown in Figs. 4 and 5. 10 gf of the contact force is applied by a spring of which spring constant is 25 gf/mm.
The friction between the surfaces of the specimen and a contactor is measured under a constant contact pressure. The contactor includes ten parallel and stacked piano steel wires such that those form the outer surface of the contactor as shown in Figs. 6 and 7. The contactor is placed on the surface of specimen. The compressional force of 50 gf by dead weight is applied to the surface of the specimen through the contactor.
In the both of the roughness and friction measurements, the specimen is moved back and forth between the interval 3 cm by a constant velocity of 0.1 cm/sec on a smooth steel plate placed horizontally where the tension of the specimen is kept 10 gf/cm (force per unit length) and the contactor is kept its position. The dimension of the plate is shown in Fig. 8. As a result, the changes of the surface friction coefficient $\mu$ and the thickness T of the specimen are obtained as shown in Figs. 9 and 10, respectively. The data obtained at the middle of the movement (i.e., from x = 0.5 cm to x = 2.5 cm) is considered for the calculation below to avoid picking up unstable data which may be generated in the transition states of the movement of the specimen.
Consequently, the average values of Surface Roughness (SR) and Coefficient of Friction (COF) are obtained from the following expressions:

$$SR = \frac{1}{2} \int_{0.5}^{2.5} | T - T' | \, dx, \qquad \text{---- (1)}$$

$$COF = \frac{1}{2} \int_{0.5}^{2.5} \mu \, dx, \qquad \text{---- (2)}$$

where $\mu$ ; frictional force/compresional force
x ; displacement of the contactor on the surface of specimen
T ; Thickness of the specimen at position x
T' ; Mean value of T
A preferred equipment for the measurements of the Surface Roughness and the Coefficient of Friction is commercially available from Kato Tech Co., Ltd., Kyoto, Japan, under the trade name "Surface Tester KES-FB4".

**Claims**

1.  A nonwoven composite laminate, comprising:

    a first nonwoven layer including component fibers containing a smoothness oil and an ethylene-propylene random copolymer which contains from 7 mol% to 15 mol% of ethylene comonomer randomly distributed in the polymer backbone; and
    an insulation layer joined to the first nonwoven layer for preventing the smoothness oil from permeating therethrough.

2.  The nonwoven composite laminate of Claim 1, wherein the smoothness oil includes a silicone oil.

3.  The nonwoven composite laminate of Claim 1, wherein the ethylene-propylene random copolymer having a PEP Ratio (determined as described in the section "Test Methods") of from 50 mol% to 100 mol%.

4.  The nonwoven composite laminate of Claim 1, wherein the component fibers are bi-component fibers which have a sheath/core structure, and the sheath contains the ethylene-propylene random copolymer.

5.  The nonwoven composite laminate of Claim 4, wherein the first nonwoven layer includes at least 10% by weight of the bi-component fibers.

6.  The nonwoven composite laminate of Claim 1, wherein the first nonwoven layer has a Coefficient of Friction (determined as described in the section "Test Methods") of less than 0.25.

7.  The nonwoven composite laminate of Claim 1, wherein the first nonwoven layer has a Surface Roughness (determined as described in the section "Test Methods") of less than about 4 $\mu$m.

8.  The nonwoven composite laminate of Claim 1, wherein the first nonwoven layer is joined to the second nonwoven layer or the insulation layer through a heat/pressure bond.

9.  A disposable article comprising the nonwoven composite laminate of Claim 1.

10. The disposable article of Claim 9, wherein the disposable article is a disposable garment including a backsheet which includes an outer cover of the nonwoven composite laminate of Claim 1 or 2.

**Patentansprüche**

1.  Vliesverbundschichtstoff, der Folgendes umfasst:

    eine erste Vliesschicht, die Komponentenfasern enthält, die ein Glättungsöl und ein statistisches Ethylen-Propylen-Copolymer enthalten, das 7 Mol% bis 15 Mol% Ethylen-Comonomer enthält, das nach dem Zufallsprinzip in der Polymer-Hauptkette verteilt ist, und
    eine Isolationsschicht, die mit der ersten Vliesschicht verbunden ist, um ein Durchdringen des Glättungsöls zu verhindern.

2.  Vliesverbundschichtstoff nach Anspruch 1, wobei das Glättungsöl ein Silikonöl enthält.

3.  Vliesverbundschichtstoff nach Anspruch 1, wobei das statistische Ethylen-Propylen-Copolymer ein nach Abschnitt "Testverfahren" ermitteltes PEP-Verhältnis von 50 Mol% bis 100 Mol% aufweist.

4.  Vliesverbundschichtstoff nach Anspruch 1, wobei die Komponentenfasern Bikomponentenfasern sind, die eine Fasermantel-/Kernstruktur aufweisen, und der Fasermantel das statistische Ethylen-Propylen-Copolymer enthält.

5.  Vliesverbundschichtstoff nach Anspruch 4, wobei die erste Vliesschicht zu mindestens 10 Gew.-% Bikomponentenfasern enthält.

6.  Vliesverbundschichtstoff nach Anspruch 1, wobei die erste Vliesschicht einen nach Abschnitt "Testverfahren" ermittelten Reibungskoeffizienten von unter 0,25 aufweist.

**7.** Vliesverbundschichtstoff nach Anspruch 1, wobei die erste Vliesschicht eine nach Abschnitt "Testverfahren" ermittelte Oberflächenrauheit von unter 4 μm aufweist.

**8.** Vliesverbundschichtstoff nach Anspruch 1, wobei die erste Vliesschicht mit der zweiten Vliesschicht oder der Isolationsschicht durch eine Wärme-/DruckVerbindung verbunden ist.

**9.** Einwegartikel, der den Vliesverbundschichtstoff nach Anspruch 1 umfasst.

**10.** Einwegartikel nach Anspruch 9, wobei der Einwegartikel ein Einwegkleidungsstück ist und eine untere Lage enthält, die eine Außenschicht des Vliesverbundschichtstoffs nach Anspruch 1 oder 2 enthält.

**Revendications**

**1.** Stratifié composite non tissé, comprenant :

une première couche de non-tissé incluant des fibres composantes contenant une huile de poli et un copolymère aléatoire éthylène-propylène qui contient de 7 % molaire à 15 % molaire de comonomère éthylène réparti de manière aléatoire dans le squelette polymère ; et
une couche d'isolation jointe à la première couche de non-tissé pour empêcher l'huile de poli de s'infiltrer à travers.

**2.** Stratifié composite non tissé selon la revendication 1, dans lequel l'huile de poli inclut une huile de silicone.

**3.** Stratifié composite non tissé selon la revendication 1, dans lequel le copolymère aléatoire éthylène-propylène ayant un rapport PEP déterminé comme décrit dans la section « Procédés de test » allant de 50 % molaire à 100 % molaire.

**4.** Stratifié composite non tissé selon la revendication 1, dans lequel les fibres composantes sont des fibres bicomposantes qui ont une structure gaine/noyau, et la gaine contient le copolymère aléatoire éthylène-propylène.

**5.** Stratifié composite non tissé selon la revendication 4, dans lequel la première couche de non-tissé inclut au moins 10 % en poids des fibres bicomposantes.

**6.** Stratifié composite non tissé selon la revendication 1, dans lequel la première couche de non-tissé a un coefficient de frottement déterminé comme décrit dans la section « Procédés de test » de moins de 0,25.

**7.** Stratifié composite non tissé selon la revendication 1, dans lequel la première couche de non-tissé a une rugosité de surface déterminée comme décrit dans la section « Procédés de test » de moins d'environ 4 μm.

**8.** Stratifié composite non tissé selon la revendication 1, dans lequel la première couche de non-tissé est jointe à la deuxième couche de non-tissé ou à la couche d'isolation via une liaison par chaleur/pression.

**9.** Article jetable comprenant le stratifié composite non tissé selon la revendication 1.

**10.** Article jetable selon la revendication 9, où l'article jetable est un vêtement jetable incluant une feuille de fond qui inclut une couverture externe du stratifié composite non tissé selon la revendication 1 ou 2.

Fig. 1

Fig. 2

Fig. 3

P=10gf

0.5

0.25R

Tl=10gf/cm

SURFACE ROUGHNESS

Fig. 4

5

SMOOTH
STEEL
(PIANO WIRE)

Fig. 5

P=50gf

5

0.5

Tl= 10gf/cm

SURFACE FRICTION

Fig. 6

5

Fig. 7

Fig. 8

Fig. 9

Fig. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5569234 A, Buell
- US 3860003 A
- US 5151092 A
- US 4610678 A
- US 4673402 A
- US 4888231 A
- US 4834735 A
- US 5190563 A
- US 5234423 A
- US 5147345 A
- EP 0797968 A1, Kurt
- US 4988344 A
- US 4988345 A
- US 3929135 A
- US 4324246 A
- US 4342314 A
- US 4463045 A
- US 5006394 A
- WO 9409736 A, Rollag
- US 4573986 A
- US 3911173 A, Sprague, Jr
- US 4785996 A, Ziecker
- US 4842666 A, Werenicz
- US 4909803 A
- US 4695278 A
- US 4795454 A
- US 4704115 A
- US 4515595 A
- US 5330458 A
- US 5167897 A, Weber
- US 5156793 A, Buell
- US 5143679 A, Weber

**Non-patent literature cited in the description**

- **M. Kakugo et al.** *Macromolecules,* 1982, vol. 15, 1150-1152